# EUROPEAN PATENT APPLICATION

(11) **EP 2 065 472 A1**
(43) Date of publication of application: **03.06.2009**
(21) Application number: 07022923.2
(22) Date of filing: 27.11.2007
(51) Int. Cl.: C12Q 1/37, G01N 33/68

(54) **Screening method for agents suitable for therapy of Alzheimer's disease**

(71) Applicant: FU Berlin, 14195 Berlin (DE)
(72) Inventor: Multhaup, Gerd, Prof., 14532 Kleinmachnow (DE); Münter, Lisa-Marie, 10437 Berlin (DE)
(74) Representative: Schüssler, Andrea

(57) **Abstract**

Described is a method of screening for therapeutic agents for the prophylaxis and therapy of Alzheimer's disease (AD), wherein agents that (i) do not interfere with the biological activity of gamma-secretase, (ii) do not exhibit cytotoxicity, (iii) lead to reduced amounts of Abeta42 and (iv) are capable of binding to an APP-GxxxG motif thereby weakening or inhibiting dimerization of the APP transmembrane sequence are suitable for prophylaxis or treatment of AD.

## Description

The present invention relates to a method of screening for therapeutic agents for the prophylaxis and therapy of Alzheimer's disease (AD), wherein agents that (i) do not interfere with the biological activity of gamma-secretase, (ii) do not exhibit cytotoxicity, (iii) lead to reduced amounts of Abeta42 and (iv) are capable of binding to an APP-GxxxG motif thereby weakening or inhibiting dimerization of the APP transmembrane sequence are suitable for prophylaxis or treatment of AD.

The deposition of amyloid beta (Abeta) in the form of senile plaques in sufficient quantity is a pathological hallmark of Alzheimer's disease (AD). Currently, Abeta is regarded as the most important diagnostic parameter, since this peptide is responsible for the plaque like extra cellular depositions (amyloidosis) in the brain which is a characteristic feature early in the course of AD. APP is a type I transmembrane (TM) protein, which undergoes proteolytic processing by several secretases. First, the bulk of the ectodomain needs to be removed by membrane-bound alpha- or beta-secretases leading to secreted forms of APP and membrane bound C-terminal fragments alpha-CTF or beta-CTF, respectively. Regulated intramembrane proteolysis (RIP) of the beta-CTF by gamma-secretase occurs only after ectodomain shedding and releases the Abeta peptide from the membrane. The active gamma-secretase complex consists of the four subunits presenilin-1 (PS-1), APH-1, PEN-2 and nicastrin. PS-1 contains two catalytic active aspartate residues in TMS-6 and TMS-7. PS-1 is endoproteolytically cleaved and the N-terminal and C-terminal fragments were shown to exist as dimers in the catalytic core of gamma-secretase. The amino termini of CTF stubs are recognized by nicastrin, which functions as a gamma-secretase substrate receptor. The gamma-secretase cleaves at variable sites thus generating Abeta peptides of varying lengths. Abeta peptides are closely linked to AD as they accumulate to amyloid plaques. Abeta42 is the pathologically most relevant form of the Abeta species since it is more prone to aggregation and found elevated in AD patient brains.

A recently discussed mechanism of gamma-secretase cleavage implies sequential proteolytic cleavage steps to release Abeta. Accordingly, the first cut occurs at the cytoplasmic edge of the TMS at the epsilon-site, i.e. residue 49 of beta-CTF. The product Abeta49 remains membrane-bound and is further processed in a sequential action mode into Abeta46 (zeta-site) and Abeta40 or Abeta42 (gamma-site). Previously it was shown that APP forms homodimers as it has been observed for other gamma-secretase substrates, e.g. the receptor tyrosine-protein kinase ErbB-4 and E-cadherin. The APP homodimerization is likely to be mediated by two different sites of the ectodomain, the loop region encompassing residues 91-111 and a second site overlapping with the collagen binding site spanning residues 448-465. Recently, a GxxxG motif could be identified mediating helix-helix interactions within the transmembrane segment of APP. This motif represents a unique homophilic interaction site of the gamma-secretase substrate CTF and controls the processing into Abeta via the final gamma-secretase cleavages. APP TMS dimerization is unique insofar as it is the only known gamma-secretase substrate with a GxxxG motif in triplicate at the start of the TMS. Any variations in APP TMS dimerization strength may fine-tune the actual preferred cleavage site by suspending the sterical hindrance. Candidate factors are nonsteroidal antiinflammatory drugs (NSAIDs) that are reported to affect the generation of different Abeta species like sulindac sulfide and flurbiprofen. Finally, it can be concluded that a strong dimerization of the substrate beta-CTF is central to AD since any events stabilizing dimerization would increase the production of Abeta42 that can easily form toxic oligomers.

Currently, only symptomatic therapies of AD are available. At best, these therapies can slow down progression of the disease, however, there is only a temporarily effect. In other words, currently available therapies (as well as therapies being based on drugs that are currently assayed in clinical trials) cannot stop worsening of the general condition of the patient and, of course, cannot lead to an improvement of the poor health. Currently available drugs are inhibitors of acetylcholine esterases, various drugs decreasing the cholesterol level, statines etc.

Thus, the technical problem underlying the present invention is to provide drugs that are useful for the causative therapy Alzheimer's disease (AD).

The solution to said technical problem is achieved by providing the embodiments characterized in the claims.

It has been found that for causative therapy of AD the formation of Abeta42 has to be inhibited. It could be shown that an increased generation of Abeta42 in the course of the disease can be caused by a strengthened stabilization of the APP transmembrane sequence via the GxxxG motif. This stabilization results in inhibition of complete degradation of the APP transmembrane sequence and partial degradation halts at a position leading to the formation of Abeta40 and Abeta42. As a result of inhibition/reduction/modulation of dimerization of the APP transmembrane sequence at the GxxxG motif of APP complete degradation can be observed. Shortened versions of Abeta species are generated that can be further catabolized in the body via usual pathways. Accordingly, the development/isolation of a drug which does not inhibit the acitivity of gamma-secretase and is capable of specifically binding to the APP GxxxG motif, thus weakening or inhibiting dimerization of the APP transmembrane sequence is desirable.

Such compound should have the following advantages:
(a) The formation of neurotoxic Abeta42 is reduced and, as a consequence, the development of AD is causatively prevented.
(b) Specificity of binding to the APP GxxxG motif or adjacent regions will selectively modulate only processing of APP but will not effect processing of other substrates of gamma-secretase.
(c) It could be demonstrated that 25% weakening of dimerization is sufficient for achieving a 50% reduction of the amount of Abeta42 (Munter et al., EMBO J. 26, 1702-1712 (2007)).

The screening method of the present invention allows isolating drugs from a pool of compounds comprising various classes of substances, i.e. drug candidates affecting the substrate/enzyme interaction of APP and gamma-secretase such that less Abeta peptide is produced. The screening method of the invention comprises two steps allowing identifying and assaying drugs having ideal characteristics. In a first step, the impact of the candidate drug on the biological activity of gamma-secretase is tested. In the second step, the capability of the drug of (a) inhibiting the formation of the neurotoxic peptide and (b) binding to the APP GxxxG motif is determined. Ideal drug candidates do not inhibit the biological activity of gamma-secretase but modify the gammma-secretase cleavage site present on APP-CTF resulting in the production of decreased amounts of Abeta42 and increased amounts a Abeta38. Such compounds can selectively bind to the APP GxxxG motif with high affinity.

### BRIEF DESCRIPTION OF THE DRAWINGS

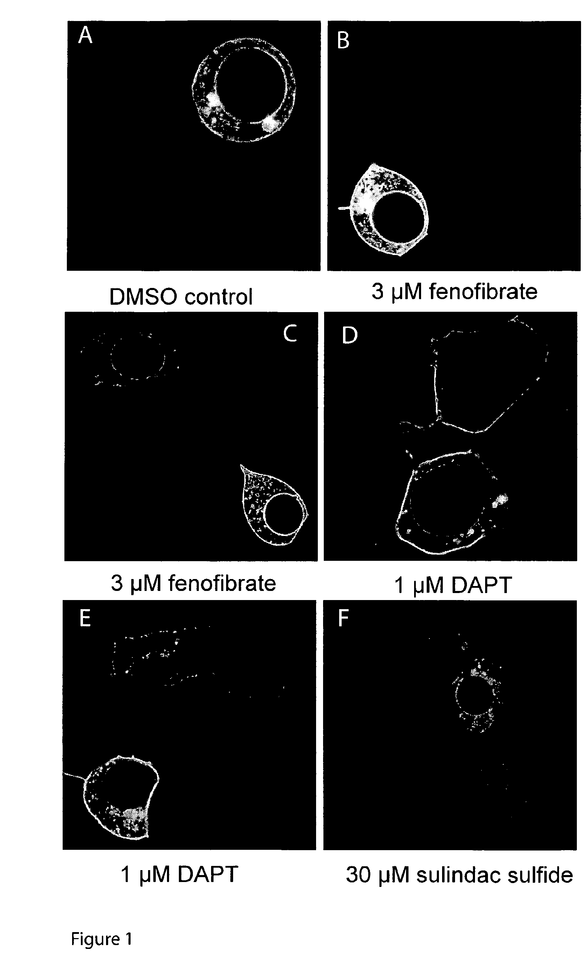

Figure 1(a): Confocal images of HEK293 cells expressing SPA4CT-YFP
   These cells express a polypeptide containing the C-terminus of APP, i.e., cleavage by beta-secretase has already occurred. Moreover, the polypeptide contains the signal peptide which is responsible for integration of the polypeptide into the cell membrane (SP = signal peptide; A4 = Abeta region; CT = C-terminus)
   **(A)** DMSO control; APP-CTF-YFP is mainly localized in the ER/nuclear membrane and the plasma membrane as well as in the Golgi apparatus. Besides APP-CTF-YFP containing cells there are many cells showing no localization of YFP, the YFP is present within the cytoplasm of the cells. These cells are very dark (see lower left corner of the image). Presumably, in these cells APP-CTF is almost completely degraded.
   **(B,C)** Addition of 3 µM Fenofibrate to the cell culture medium. More cells showing a clear localization of APP-CTF within the cell membrane and the ER/nuclear membrane are present.
   **(D,E)** Addition of 1 µM DAPT to the cell culture medium. APP-CTF preferably accumulates at the plasma membrane, the ER/nuclear membrane shows comparatively low fluorescence. The ER/Golgi is well visible.
   **(F)** Addition of 30 µM sulindac sulfide. APP-CTF preferably accumulates at the ER/nuclear membrane. The plasma membrane shows comparatively weak staining.

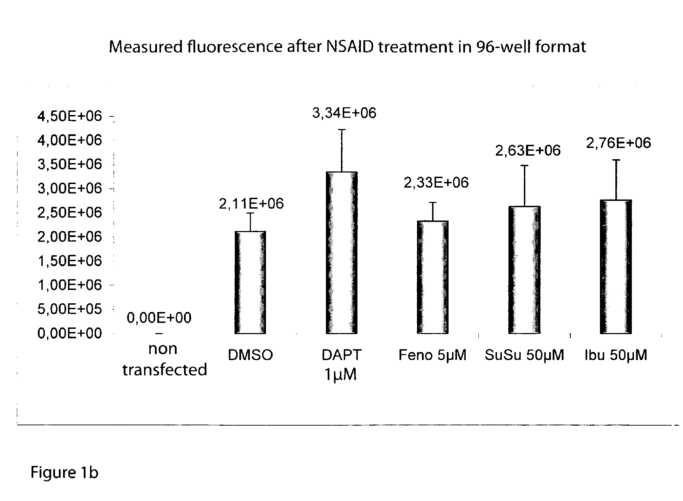

Figure 1(b): Quantification of gamma-secretase inhibition
   Average fluorescence of HEK293 cells expressing APP-CTF-YFP (substrate of gamma-secretase) was determined. Cells were either untransfected (no fluorescence, negative control) or treated with DMSO (vehicle control), 1 µM DAPT (gamma-secretase inhibitor, positive control) or different NSAIDs, i.e. 5 µM Fenofibrate (Feno), 50 µM Sulindac Sulfide (SuSu), 50 µM Ibuprofen (Ibu). Compared to the DMSO control, DAPT treatments leads to increased fluorescence due to an inhibition of the degradation of the gamma-secretase substrate, as expected. The treatment with NSAIDs leads to a slight increase of fluorescence, indicating that at certain high concentrations, the tested NSAIDs are able to reduce gamma-secretase activity.

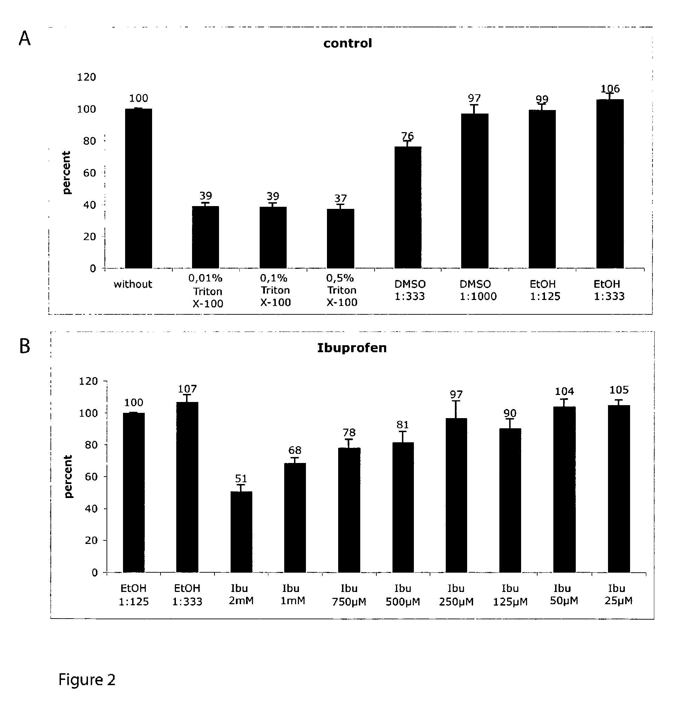

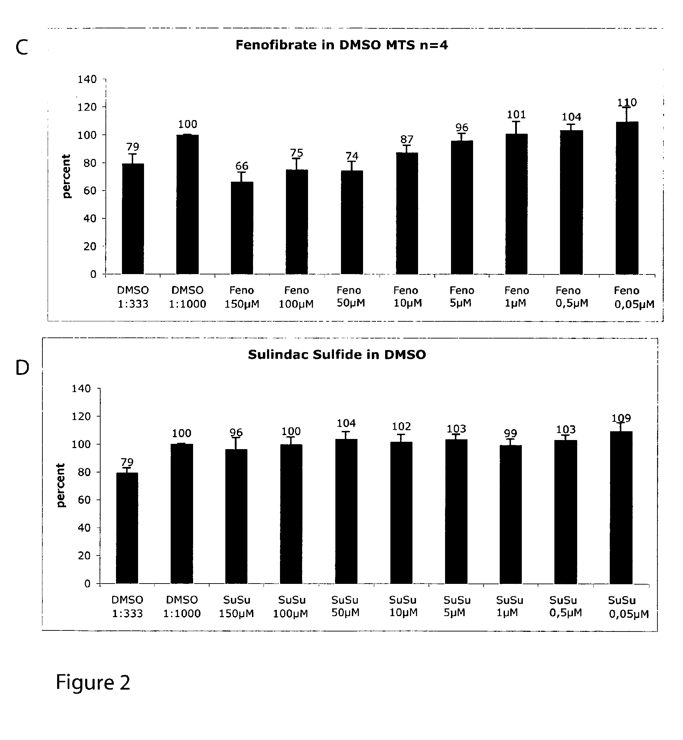

Figure 2: MTS assay, assay of viable SH-SY5Y cells for the determination of the cytotoxicity of various compounds, i.e., various NSAIDs
   **(A)** MTS assay of untreated (without, as a negative control), with triton treated (as a positive control), DMSO or ethanol (EtOH) treated cells. When diluted 1:1000 or more, DMSO is no longer toxic and when diluted 1:250 or more, EtOH is no longer toxic.
   **(B)** MTS assay of cells treated with ibuprofen. At a concentration of 250 µM ibuprofen is no longer toxic.
   **(C)** MTS assay of cells treated with Fenofibrate. At a concentration of 5 µM Fenofibrate is no longer toxic.
   **(D)** MTS assay of cells treated with sulindac sulfide. At a concentration of 150 µM sulindac sulfide is no longer toxic.

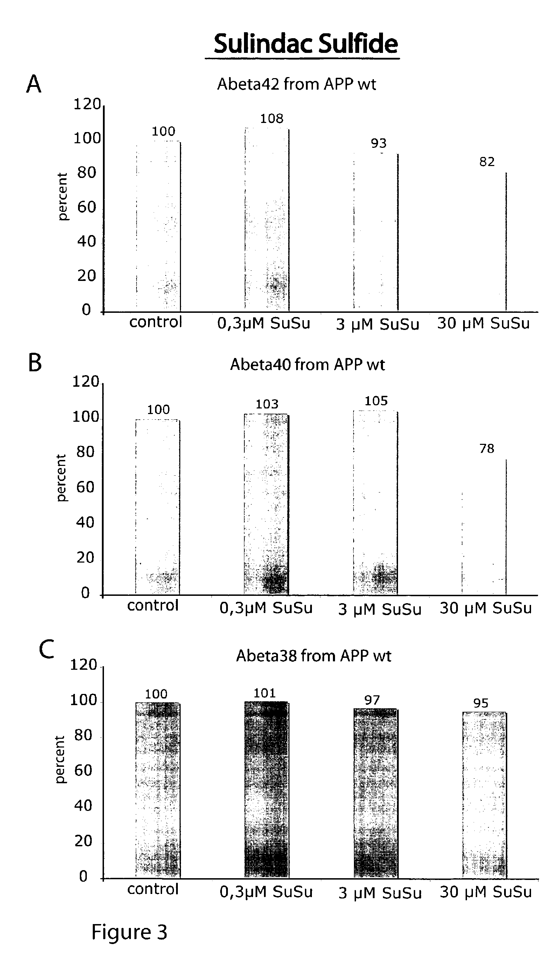

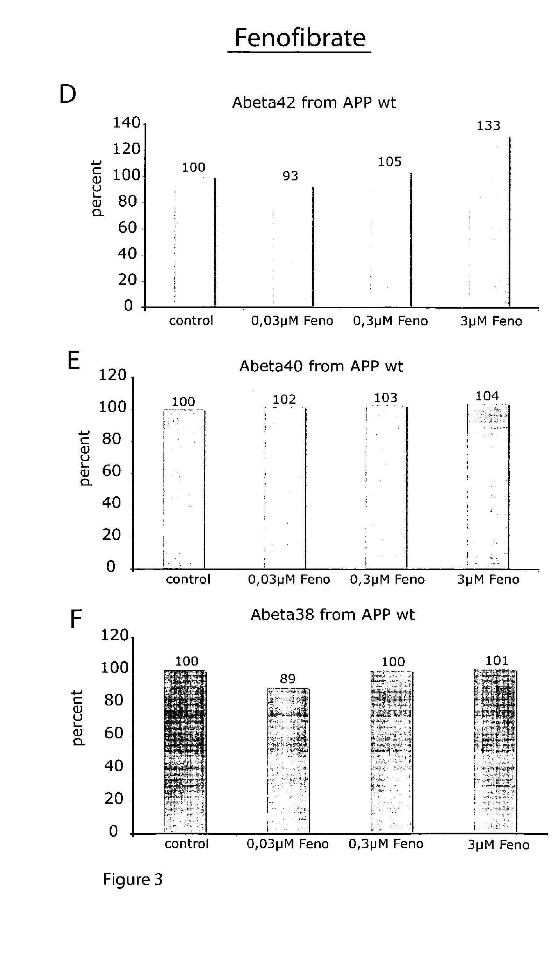

Figure 3: Quantification of Abeta species secreted from SH-SY5Y cells which stably express wild type APP
   Sulindac sulfide decreases in a concentration dependent manner the secreted amount of Abeta42 (A) and its impact on the amounts of Abeta40 (B) and Abeta38 (C) is low. Fenofibrate increases in a concentration dependent manner the secreted amount of Abeta42 (D) and its impact on the amounts of Abeta40 **(E)** and Abeta38 (F) is also low.
Figure 4: MALDI-MS analyses of secreted Abeta species after treatment with NAIDs
   After treatment with NSAIDs various Abeta species can be detected. The correlation of the signal intensities indicates a shift of the cleavage sites of gamma-secretase and, thus, the production of Abeta species having varying lengths.
Figure 5: Impact of various compounds on the dimerization of transmembrane sequence containing peptides regions
   **(A)** Addition of sulindac sulfide weakens the stability of dimerization of the transmembrane sequence of Abeta29-42 in a concentration dependent manner. As a control, the solvent ethanol (EtOH) is used.
   **(B)** Ibuprofen weakens the strength of dimerization of the transmembrane sequence Abeta29-42.
   **(C)** Fenofibrate clearly enhances the stability of the dimer of the transmembrane sequence Abeta29-42 wt. The impact on the altered transmembrane sequence Abeta29-42 G29/33A is comparatively less pronounced. Thus, it can be indirectly concluded that for stabilization of the dimer by fenofibrate the presence of the GxxxG motif of the transmembrane sequence is required.

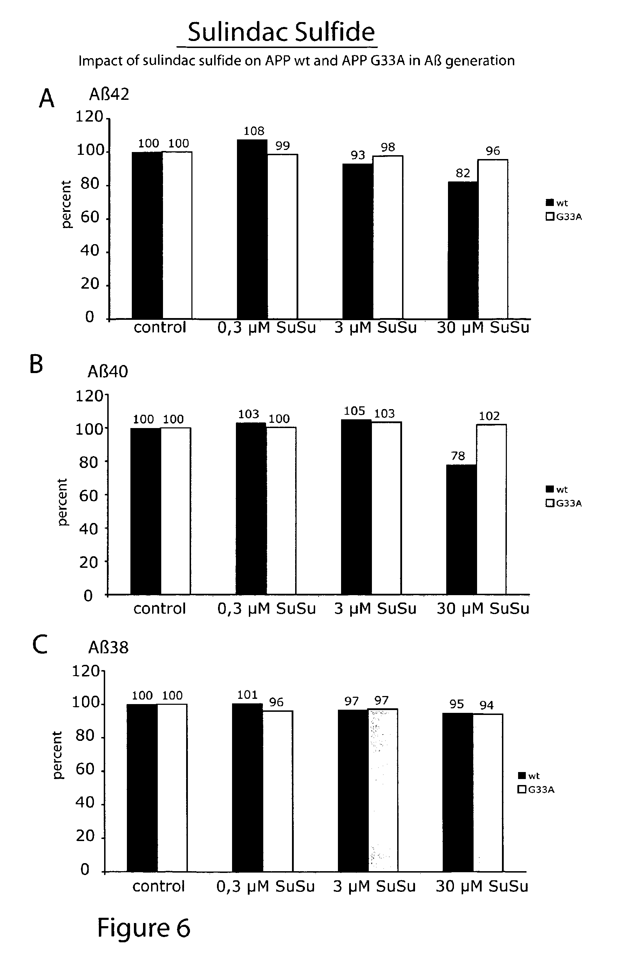

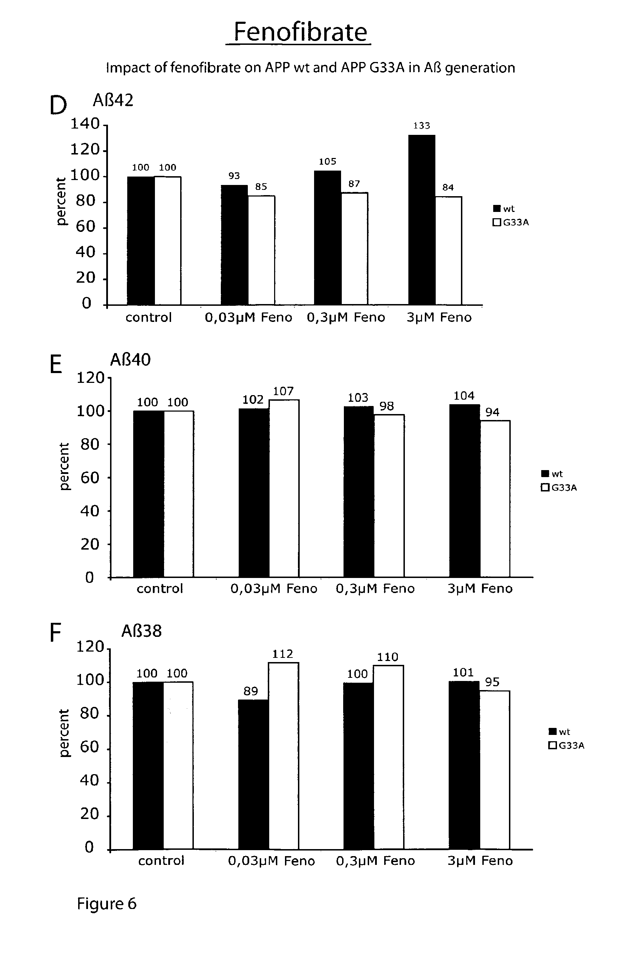

Figure 6: Impact of various compounds on the formation of Abeta from APP-GxxxG mutants
   Comparison of the data of Figure 3 relating to APP wt transfected cells with data of APP G33A-transfected cells.
   **(A)** Sulindac sulfide reduces the secreted amount of Abeta42 from APP wt, but has no impact on the amount of Abeta42 from APP G33A.
   **(B,C)** Sulindac sulfide does not substantially reduce the amounts of Abeta40 (B) and Abeta38 (C) from APP wt- or APP G33A-transfected cells.
   **(D)** Fenofibrate increases the amount of Abeta42 from APP wt-transfected cells, but not from APP G33A-transfected cells.
   **(E,F)** Fenofibrate does not substantially reduce the amounts of Abeta40 (B) and Abeta38 (C) from APP wt- or APP G33A-transfected cells.

The present invention, thus, provides a method of screening for a therapeutic agent useful in the prophylaxis or treatment of AD comprising the steps of
(a) determining whether a test compound interferes with the biological activity of gamma-secretase;
(b) determining the cytotoxic activity of said test compound of (a);
(c) determining the amount of Abeta42 and, preferably, shorter Abeta species generated in the presence of said test compound; and
(d) contacting said test compound with a peptide comprising an APP-GxxxG motif and determining whether said test compound is capable of binding to said motif
   wherein (i) lack of interference with the biological acivity of gamma-secretase, (ii) substantial lack of cytotoxicity, (iii) reduction of the amounts of Abeta42, and (iv) binding to the APP-GxxxG motif thereby weakening or inhibiting dimerization of the APP-GxxxG containing peptide indicates that said test compound might be suitable for prophylaxis or treatment of AD.

The chronological order of steps (a) to (d) can be any order, e.g., step (b) can be carried out as a first step etc.

The test compounds may be very different compounds, both naturally occurring compounds and synthetic, organic and inorganic compounds as well as polymers (e.g. oligopeptides, polypeptides, oligonucleotides and polynucleotides) as well as small molecules, antibodies, sugar, fatty acids, nucleotides and nucleotide analogs, analogs of naturally occurring structures (e.g. peptide "imitators", nucleic acid analogs, etc.) and numerous other compounds. Test compounds can also be screened on a large scale, e.g. by screening a very large number being able to contain synthetic or natural molecules. Thus, the test compound of a preferred embodiment of the method according to the invention forms part of a substance library.

A large number of possibly useful test compounds can be screened in extracts of natural products as a starting material. Such extracts may be derived from a large number of sources, e.g. the following species: fungi, actinomycetes, algae, insects, protozoa, plants and bacteria. The extracts showing activity can then be analyzed for isolating the active molecule.

In a preferred embodiment of the screening method of the present invention, the test compound is from a library of fungal extracts. A particularly preferred library of fungal extracts is from a *Penicillium* or *Aspergillus* species. Alternatively, the library is a library of NSAIDs-like compounds.

The assay can be carried out in a cell-free extract or cell lysate or in vivo using suitable cell lines. Examples of cells useful for the first step of the screening method of the present invention are human (SH-SY5Y), monkey, rat mammalian cell lines or yeast cells expressing all four proteins necessary for gamma-secretase activity. Examples of cells useful for the second step of the screening method of the present invention are mammalian cells, preferably human cells, and cytotoxicity can be determined by routine methods. Examples of cells useful for the third step of the screening method of the present invention are human (SH-SY5Y), monkey, rat mammalian cell lines or yeast cells expressing APP-CTF, APP or APPsw. Examples of cells useful for the fourth step of the screening method of the present invention are any bacterial cells containing any reporter gene (or measuring directly FRET via CFP and YFP), e.g., under the control of the ctx ToxR-transcription activator promoter like, e.g., *E. coli* FHK12. A similar assay could be performed in eukaryotic cells using a fusion construct composed of the TMS and, e.g., an intracellular tyrosine kinase activity. The dimeric state would be reported by the degree of auto-phosphorylation by the tyrosine kinase.

The test compound can be added to the cells via the medium, for example. Fundamentally suited assay formats for identifying positive test compounds are well known in the biotechnological and pharmaceutical industries and additional assays and variations of the above assays provided for the purpose of illustration are obvious to the person skilled in the art. The person skilled in the art can, e.g., draw on the assay formats given in the examples, below.

The screening method according to the invention can be modified by means of protocols described in scientific literature and patent literature. For example, a large number of possibly useful molecules can be screened in a single test. For example, when a field of 1000 compounds shall be screened, in principle all of the 1000 compounds can be placed in a microtitration plate well and tested at the same time. The pool of 1000 can be divided into 10 pools of 100 and the process can be repeated until an individual positive test compound is identified. In any case, the production and simultaneous screening of large libraries from synthetic molecules can be carried out by means of well known methods of combinatorial chemistry.

Compounds isolated by the screening method of the invention are suitable as therapeutics to treat, prevent, control, or lessen the severity of AD, or can be used as lead compounds for the development of drugs that have additional desired properties.

In a preferred embodiment of the screening method of the present invention, steps (a) and/or (c) are carried out by adding the test compound to a cell culture (or, alternatively, a cell lysate). Suitable cells are known to the person skilled in the art and easily available. Examples of suitable cells are higher eucaryotic cells expressing substrates, e.g., SH-SY5Y, HEK293 etc. In further preferred embodiments, the cells are continuous mammalian cell lines including, without limitation, human, monkey or rat cells. The cells can include exogenous (a) APP-CTF, APP or APPsw and/or (b) gamma-secretase encoding DNA (i.e., DNA artificially introduced into the cells, e.g., by transfection, infection, transformation etc.) effecting expression of APP-CTF, APP, APPsw and/or gamma-secretase, preferably over expression in comparison to the wild type cells without such exogenous DNA.

In a more preferred embodiment of the method of the invention APP-CTF is present as a fusion protein. The fusion partner, i.e., second polypeptide, can be a polypeptide being capable of generating a detectable signal. The second polypeptide segment of a fusion protein comprising a reporter protein can be a full-length protein or a protein fragment. Proteins commonly used in fusion protein construction include, but are not limited to beta-galactosidase, beta-glucuronidase, green fluorescent protein (GFP), YFP, autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions.

In an even more preferred embodiment of the method of the present invention, the fusion partner of the fusion protein is a fluorescent protein. Examples of suitable fluorescent proteins are already described above.

In a further preferred embodiment of the method of the present invention, the second step (b) is carried out by adding the test compound to a neuronal cell culture and cytotoxicity is determined by use of a proliferation or cell viability assay. Examples of preferred neuronal cells are SH-SY5Y cells. Preferably, cytotoxicity is determined by using an MTS or MTT assay.

In a further preferred embodiment of the screening method of the present invention, the third step (c) is carried out by assaying whether the test compound leads to reduced amounts of Abeta42 and, in a preferred embodiment, to increased amounts of shorter Abeta species. Promising drugs are compounds leading to reduced amounts of Abeta42 and increased amounts of shorter Abeta species like the gamma-secretase cleavage products Abeta40, Abeta38 and Abeta35.

The person skilled in the art is familiar with suitable methods for checking the amounts of the various Abeta species. Preferably, detection and/or quantification of Abeta species is accomplished by immunological methods using specific antibodies or fragments thereof. The term "antibody" as used herein, preferably, relates to antibodies which consist essentially of pooled monoclonal antibodies with different epitopic specificities, as well as distinct monoclonal antibody preparations. Monoclonal antibodies are made from an antigen containing fragments of the Abeta peptides by methods well known to those skilled in the art (see, e.g., Köhler et al., Nature 256 (1975), 495). As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody.

The antibodies can be detectably labelled, for example, with a radioisotope, a bioluminescent compound, a chemiluminescent compound, a fluorescent compound, a metal chelate, or an enzyme (e.g. horse-radish peroxidase, alkaline phosphatase, beta-galactosidase, malate dehydrogenase, glucose oxidase, urease, catalase etc.) which, in turn, when later exposed to a substrate will react to the substrate in such a manner as to produce a chemical moiety which can be detected. The antibodies can also be immobilized on an insoluble carrier, e.g. glass, polystyrene, polypropylene, polyethylene, dextran, nylon, natural and modified celluloses, polyacrylamides, agarose and magnetic beads.

Any of a wide range of well known immunodetection techniques can be used. Examples of immunoassays suitable for the method of the present invention are competitive or sandwich assays, such as the enzyme linked immunosorbent assay (ELISA), the radioimmunoassay (RIA) or Western Blots. Suitable antibody assay labels are known in the art and include enzyme labels, such as, glucose oxidase, and radioisotopes, such as iodine (¹²⁵I, ¹²¹I), carbon (¹⁴C) , sulphur (³⁵S) , tritium (³H), indium (¹¹²In), and technetium (⁹⁹mTc) , and fluorescent labels, such as fluorescein and rhodamine.

Preferred techniques are ELISA and immunoprecipitation. Detection and/or quantification can also be carried out by MALDI-MS analysis.

In a further preferred embodiment of the screening method of the invention, the binding in step (d) is determined by measuring the decrease of dimerization in a bacterial test system. Any reporter gene system, e.g., a reporter system according to the ToxR principle is, suitable for this assay step. For example, the reduction of FRET efficiency (using a fusion protein tagged with, e.g., YFP and CFP) can be determined. Alternatively, an ELISA based assay with, e.g., synthetic peptides can be applied where dimerization is reduced with increasing amounts of binding compounds (competition between dimers and binding compounds).

In a more preferred embodiment, the bacterial test system is the ToxR system.

In an even more preferred embodiment, the peptide of step (d) comprising an APP-GxxxG motif is present as a fusion protein comprising a detectable moiety. Preferably, said detectable moiety is a YFP- or CFP-tag.

In a particularly preferred embodiment of the screening method of the invention, the peptide comprising an APP-GxxxG motif is a peptide consisting of the transmembrane sequence of APP with amino acid residues 29-42 of Abeta (GAIIGLMVGGVVIA). Preferably, in step (d) the specificity of binding is determined by using at least one peptide comprising a mutated GxxxG motif as a negative control (e.g., with the GxxxG-transmembrane sequence as part of the ToxR- fusion protein).

The following examples illustrate the invention.

### Example 1

### General Methods

### Plasmids and transfections

Generation of ToxR-system plasmids has been described (Langosch et al., J Mol Biol 263 (1996), 525-30). APP695 with N-terminal Myc-tag and C-terminal Flag-tag, SPA4CT with C-terminal Flag-tag or SPA4CT with C-terminal YFP-tag were used as a template to introduce GxxxG mutations by site-directed mutagenesis. The cDNAs were inserted into pCEP4 (Invitrogen, Karlsruhe, Germany) or in pcDNA3 (Invitrogen), which contain a hygromycine-resistance or geneticine-resistance gene. For stable or transient expression, plasmids were transfected into SH-SY5Y or HEK293 cell lines (ATCC number: CRL-2266 and CRL-1573, respectively) using Transfectine (Bio-Rad, München, Germany) following the manufacturer's instructions. All sequences were confirmed by dideoxy sequencing.

Treatment of fluorescent cells with NSAIDs HEK293 cells were transiently transfected with SPA4CT-YFP. After 24 h, cells were treated with varying concentrations (indicated in the figures) of different NSAIDs, gamma-secretase inhibitor N-[N-(3,5-difluorophenacetyl-L-alanyl)]-S-phenylglycine *t*-butyl ester (DAPT) or vehicle control for 6 h. The subcellular distribution of APP-CTF-YFP within living cells was analyzed by confocal laser scanning microscopy (LSM) .

To quantify the effects of NSAIDs on gamma-secretase activity, HEK293 expressing SPA4CT-YFP were spread into a 96-well dish and were treated with NSAIDs or controls for 6 h. Intensity of YFP fluorescence was quantified with adjusted filters in a 96-well fluorescence reader (company BertholdTech, Mithras).

### Cytotoxicity

SH-SY5Y cells expressing SPA4CT wt were spread at a density of 30.000 cells per 96-well. The cell culture media (OptiMEM, Invitrogen or serum containing medium, Biochrom) were supplemented with NSAIDs in varying concentrations as well as positive controls (without treatment or vehicle control) and negative controls (0.01% to 0.5% Triton X-100). Each well was incubated with 100 µl of supplemented media for 24 h. Then, 20 µl of the MTS stain solution (Promega, cell viability assay) was added and after 2-4 h incubation time the absorbance at 562 nm was measured.

Quantification of Abeta 42, Abeta 40 and Abeta38 with ELISA Stably transfected cells were plated at a density of 2.8 x 10⁵ cells per 12-well. The day after splitting, 200 µl of fresh media supplemented with NSAIDs or control vehicles were added and incubated for 6 h. For Abeta40 and Abeta42-specific ELISAs, 50 µl of media were analyzed according to the manufacturer's instruction (The Genetics company (TGC), Basel, Switzerland). The same protocol was applied to determine Abeta38 levels, except that the antibody BAP-29 (provided by M. Brockhaus, Roche, Basel, Switzerland) was used.

### MALDI-Mass spectrometry

Stably transfected cells were plated at a density of 2 x 10⁶ cells per 60-mm dish and treated with supplemented serum-containing media (Biochrom) or OptiMEM (Invitrogen). Abeta from conditioned media was immunoprecipitated with the antibody Wo-2 and Protein-G-Sepharose. The Sepharose was washed twice in PBS and twice in 100 mM ammonium chloride, pH 7.4. Abeta was eluted twice with 300 µl of 50% acetic acid and the eluate was vacuum-dried. MALDI-MS analysis was carried out on sinapinic acid matrix with an UltraflexII TOF/TOF (Bruker Daltonics, Bremen, Germany.)

### ToxR-system

*E.* coli FHK12 cells were transformed with accordant DNA constructs encoding for ToxR-fusionproteins containing at least a part of the APP transmembrane sequence (e.g. sequence Abeta 29-42). Cells were grown in either FHK medium or minimal medium (e.g. M9 medium) supplemented with NSAIDs and corresponding vehicle controls for 6h. Then, aliquots of the cells were lysed and beta-galactosidase activity was measured (Langosch et al., 1996).

### Example 2

### Isolation of drugs for therapy of AD

### (A) First screening step: Identification of compounds by use of a cell culture system which are capable of reducing the amount of Abeta42 but do not inhibit the enzymatic activity of gamma-secretase

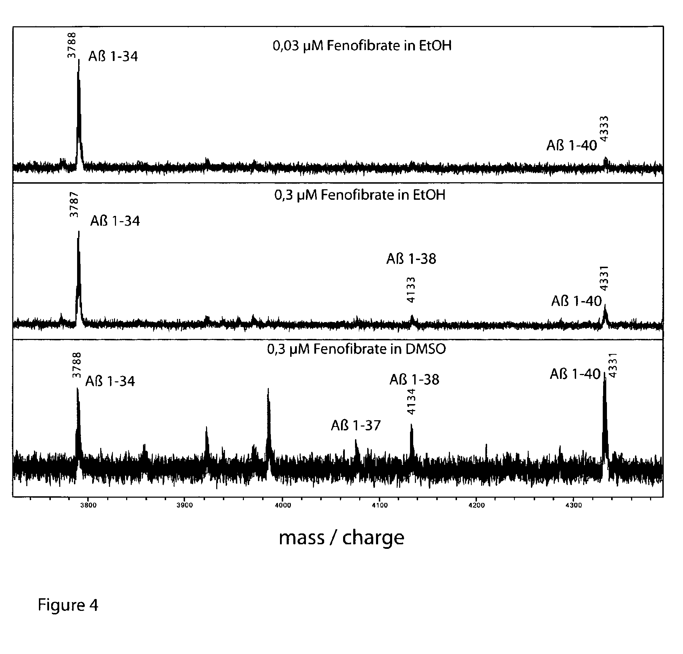

(i) Cells expressing a fusion protein comprising the substrate of gamma-secretase (APP-CTF) and a fluorescent protein (FP) are incubated with the drug candidate in a 96 well plate. Prior to addition to the cells, the drug is dissolved in EtOH or DMSO. Controls are (1) untreated culture medium, (2) culture medium containing EtOH or DMSO, and (3) culture medium with known inhibitors of gamma-secretase. After incubation for 6h to 24 hours the fluorescence is determined. If gamma-secretase is active, the fusion protein is degraded and the fluorescent protein spreads in the cell, e.g., the cell cytoplasm. As a consequence, intensive fluorescence is not detectable. If the gamma-secretase is inhibited, the substrate of gamma-secretase accumulates at the plasma membrane resulting in an intensive (and traceable) fluorescence (see, for further experimental details and results Figure 1 and 1.II and the figure legend). Drug candidates which inhibit the biological activity (i.e. cleavage of the substrate) of gamma-secretase are discarded.
(ii) Initially, the cytotoxicity of the compounds in cultures of neuronal cells is determined using a cell proliferation assay or an assay based on viable cells (Figure 2). Then, APP-CTF, APP or APPsw (APPsw: APP with the familial AD mutation "swedish" (K670N M671L); APP-CTF: APP-C-terminal fragment; CTF: after alpha- or beta-secretase cleavage) expressing cells are incubated with the drug candidate (two different concentrations, 96 well plate format). After incubation for 6 to 24h the amount of Abeta42 secreted from the cell culture medium is determined using ELISA (see, for further experimental details and results Figure 3 and the figure legend). Drug candidates causing a reduction of the amount of Abeta42 are assayed in a further step comprising ELISA and MALDI-MS. First, the amounts of Abeta40 and Abeta38 (as well as additional possible Abeta species) are quantified by use of ELISA. Then, the entire spectrum of secreted Abeta species is assayed using immunoprecipitation and MALDI-MS analysis (see, for further experimental details and results Figure 4 and the figure legend). Compounds leading to reduced amounts of Abeta42 and increased amounts of shorter Abeta species are selected and assayed in a further screening step.

### (B) Second screening step using a bacterial system and determination of the effect via the substrate, i.e., binding to the GxxxG motif of TMS of APP or adjacent regions and impairment of dimerization

The second screening step is carried out in order to find compounds selectively modulating only the processing of APP via binding to the transmembrane sequence (TMS) of APP. The APP-GxxxG motif is, inter alia, a perfect target structure for selecting compounds characterized by high affinity binding (Munter et al., EMBO J. 26 (2007), 1702-12). In the bacterial assay system (ToxR system) only the transmembrane sequence of APP containing the amino acid residues 29-42 of the Abeta region (GAIIGLMVGGVVIA) is expressed as part of a fusion protein. This part of the sequence causes dimerization of the fusion proteins and, as a consequence, transcription of a reporter gene (Munter et al., 2007). The rate of transcription correlates with the degree of dimerization and can be determined by measuring the conversion of a coloured substrate. The compounds and adequate controls are added to the culture medium. After incubation for 2 to 24h the degree of dimerization is determined. Binding of compounds to the amino acid sequence Abeta 29-42 results in weakening of dimerization and this is determined by measuring the decrease of enzymatic activity. Assays containing various single mutations of the Abeta sequence are used as controls: It is expected that the drug candidate will not bind to such sequences and, thus, not inhibit dimerization. As a further control, "foreign sequences", e.g., the transmembrane sequence of "Coxsackie virus and adenoviral receptors" are used.

### Example 3

### Determination of the effect of NSAIDs using the ToxR system

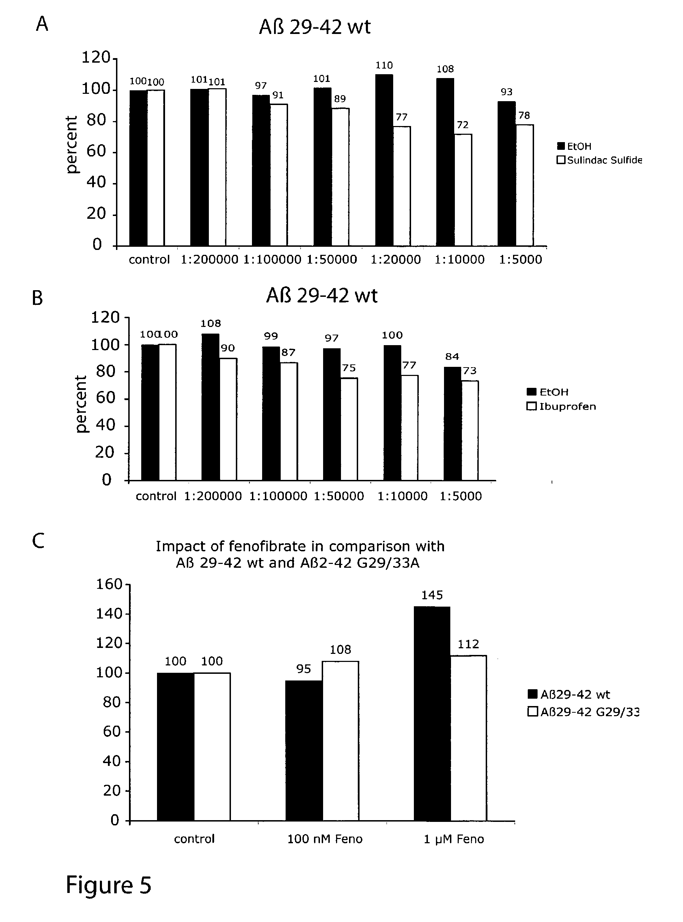

The effect of the drugs on the dimerization motif GxxxG of TMS of APP is assayed. Drug candidates were added to the cell culture medium containing APP-CTF, APP or APPsw expressing neuronal cells or cells containing the APP-processing machinery as described in Excample 2. In addition, cell lines were assayed expressing altered APP molecules characterized by a mutated GxxxG motif. Binding of the compounds to the APP GxxxG motif results in an altered processing only of the wild type proteins, but not the altered GxxxG motif of mutants (see, for further experimental details and results Figure 5 and the figure legend).

### Example 4

### Comparison of the effect of sulindac sulphide and Fenofibrate on APP-wt and APP-G33A

Detection and characterization of the binding of the drug candidate to the GxxxG motif. Binding of the compounds to Abeta42 is verified by use of BIACORE and the K_{D}-value is determined. Binding of the compounds to the GAIIG motif should lead to lack of binding to corresponding Abeta42-fragments or GxxxG mutant peptides. Similarly, using ELISA with synthetical peptides binding of NSAIDs can be determined via a competition assay (see, for further experimental details and results Figure 6 and the figure legend).

## Claims

1. A method of screening for a therapeutic agent useful in the prophylaxis or treatment of Alzheimer Disease (AD) comprising the steps of
(a) determining whether a test compound interferes with the biological activity of gamma-secretase;
(b) determining the cytotoxic activity of said test compound of (a) ;
(c) determining the amount of Abeta42 and, preferably, shorter Abeta species generated in the presence of said test compound; and
(d) contacting said test compound with a peptide comprising an APP-GxxxG motif and determining whether said test compound is capable of binding to said motif
wherein (i) lack of interference with the biological acivity of gamma-secretase, (ii) substantial lack of cytotoxicity, (iii) reduction of the amounts of Abeta42, and (iv) binding to the APP-GxxxG motif thereby weakening or inhibiting dimerization of the APP-GxxxG containing peptide indicates that said test compound might be suitable for prophylaxis or treatment of AD.

2. The method of claim 1, wherein step (a) is carried out by adding the test compound to a cell culture comprising cells which express (i) gamma-secretase and (ii) APP-CTF.

3. The method of claim 2, wherein APP-CTF is present as a fusion protein.

4. The method of claim 3, wherein the fusion partner of the fusion protein is a fluorescent protein.

5. The method of claim 4, wherein the fluorescent protein is YFP or CFP.

6. The method of any one of claims 1 to 5, wherein step (b) is carried out by adding the test compound to a neuronal cell culture and inhibition of proliferation or cytotoxcicity is determined.

7. The method of claim 6, wherein cell viability is determined by an MTS assay.

8. The method of any one of claims 1 to 7, wherein in step (c) test compounds leading to reduced amounts of Abeta42 and increased amounts of shorter Abeta species are selected.

9. The method of any one of claims 1 to 8, wherein in step (d) binding is determined by measuring the decrease of dimerization in a bacterial test system.

10. The method of claim 9, wherein the bacterial test system is the ToxR system.

11. The method of any one of claims 1 to 10, wherein the peptide of step (d) comprising an APP-GxxxG motif is present as a fusion protein comprising a detectable moiety.

12. The method of any one of claims 1 to 11, wherein the peptide comprising an APP-GxxxG motif is a peptide consisting of the transmembrane sequence of APP with amino acid residues 29-42 of Abeta (GAIIGLMVGGVVIA).

13. The method of any one of claims 1 to 12, wherein in step (d) the specificity of binding is determined by using at least one peptide comprising a mutated GxxxG motif as a negative control.
